# EUROPEAN PATENT SPECIFICATION

(11) **EP 3 512 505 B1**
(45) Date of publication and mention of the grant of the patent: **15.03.2023**
(21) Application number: 17768140.0
(22) Date of filing: 18.09.2017
(51) Int. Cl.: A61K 31/40, A61K 9/20

(54) **VILDAGLIPTIN PHARMACEUTICAL COMPOSITIONS**
PHARMAZEUTISCHE ZUSAMMENSETZUNGEN VON VILDAGLIPTIN
COMPOSITIONS PHARMACEUTIQUES DE VILDAGLIPTINE

(30) Priority: 16.09.2016 EP 16189135; 15.09.2017 ES 201731122
(43) Date of publication of application: 24.07.2019
(73) Proprietor: Galenicum Health S.L.U., 08950 Esplugues de Llobregat (ES)
(72) Inventor: ARROYO HIDALGO, Sergio, 08005 Barcelona (ES); PLADEVALL ROSES, Mireia, 08005 Barcelona (ES); MIGUEZ DIEZ, Eric, 08005 Barcelona (ES)
(74) Representative: Torrejón-Nieto, Javier
(86) International application number: PCT/EP2017/073492
(87) International publication number: WO 2018/050892

(56) References cited:
- EP-A1- 2 468 361
- WO-A1-2010/036600
- WO-A1-2014/101986
- WO-A1-2015/097234
- US-A1- 2011 086 096

## Description

The present invention relates to stable pharmaceutical compositions of vildagliptin and to a process for the manufacture of said stable pharmaceutical compositions as defined by the claims. Also disclosed but not claimed are uniform pharmaceutical batches of said compositions.

### STATE OF THE ART

Type 2 diabetes is a chronic and progressive disease arising from a complex pathophysiology involving the dual endocrine defects of insulin resistance and impaired insulin secretion. The treatment of type 2 diabetes typically begins with diet and exercise, followed by oral antidiabetic monotherapy. For many patients, these regimens do not sufficiently control glycemia during long-term treatment, leading to a requirement for combination therapy within several years following diagnosis. Dipeptidyl dipeptidase-IV (DPP-IV), also known as adenosine deaminase complexing protein 2 or CD26, is an antigenic enzyme expressed on the surface of most cell types and is associated with immune regulation, signal transduction and apoptosis. DPP-IV plays a major role in glucose metabolism. It is responsible for the degradation of incretins such as GLP-1. A new class of oral hypoglycemics called DPP-IV inhibitors work by inhibiting the action of this enzyme, thereby prolonging incretin effect in vivo. They can be used to treat diabetes mellitus type 2. Glucagon increases blood glucose levels, and DPP-IV inhibitors reduce glucagon and blood glucose levels. The mechanism of DPP-IV inhibitors is to increase incretin levels (GLP-1 and GIP), which inhibit glucagon release, which in turn increases insulin secretion, decreases gastric emptying, and decreases blood glucose levels.

Vildagliptin is a DPP-IV inhibitor developed for the treatment of type 2 diabetes. Vildagliptin, also called LAF-237, is disclosed in WO0034241. The chemical name of vildagliptin is (2S)-1 -[2-[(3-hydroxy-1 -adamantyl)amino]acetyl]pyrrolidine-2-carbonitrile. The empirical formula of vildagliptin is C₁₇H₂₅N₃O₂ and the compound has a molecular weight of 303.4 g/mol. The structural formula of vildagliptin is (I): 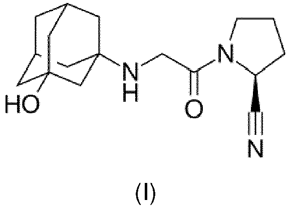 Vildagliptin is commercially available as 50 mg tablets under the brand name Galvus^{®} by Novartis, which is indicated for the treatment of type 2 diabetes. Vildagliptin is a moisture sensitive molecule. Novartis patent WO0034241 discloses vildagliptin tablets manufactured by alcoholic moisture granulation; however non-aqueous granulation is an expensive procedure which also has the problem of ensuring the elimination of the organic solvent employed. Also by Novartis WO2005067976 and WO2006078593 relate to vildagliptin tablets manufactured by direct compression, however direct compression method can give rise to segregation and lack of uniformity problems, as well as compression issues. Novartis' WO2006021455 relates to vildagliptin tablets manufactured by melt granulation. Melt granulation is an expensive procedure since it requires high input of energy and is not suitable for heat sensitive material because of the elevated temperature involved. EP2468361 relates to vildagliptin granules which are coated with pullulan or polyvinyl alcohol. WO2015097234 discloses tablets comprising vildagliptin and metformin, wherein linagliptin is granulated by different techniques. WO2014101986 discloses tablets comprising vildagliptin manufactured by dry granulation aiming to improve flowability and compressibility of vildagliptin granules. US201108096 is directed to sustained release formulations of vildagliptin and discloses vildagliptin tablets prepared by dry granulation methods. It would be desirable to provide stable vildagliptin formulations which do not require granulation with organic solvents, melt granulation or coated granules and still provide good chemical stability and physical properties.

### SUMMARY OF THE INVENTION

The present invention provides a more stable pharmaceutical composition of vildagliptin than those in the market. The pharmaceutical compositions as disclosed herein have smaller amounts of impurities originated from the degradation of vildagliptin. Also, the pharmaceutical compositions of the present invention can be easily manufactured by dry techniques, surprisingly resulting in a more stable, uniform, immediate release vildagliptin composition and improved dissolution profile.

The present invention provides a pharmaceutical composition comprising granulated vildagliptin which can be manufactured in the absence of a liquid, as defined by the claims. In this way, the pharmaceutical compositions of the present invention have a lower water content and show a better stability than the prior art compositions and good compaction. Surprisingly, the batches of the pharmaceutical compositions of the present invention have very good content uniformity, even without performing a wet granulation and in spite of the low concentration of the active agent in said pharmaceutical compositions.

### DETAILED DESCRIPTION OF THE INVENTION

In a first aspect, the present invention relates to a tablet which comprises at least the following components as defined by the claims:
(a) a granulate which comprises at least a polyol and vildagliptin or a pharmaceutically acceptable salt thereof; and
(b) an extragranular component.

The water content of the tablet is less than 10% w/w in respect of the total weight of the tablet, when measured by Karl Fischer titration.

As used herein, the term "granulate" or "granulate component" or "intragranular component" refers to a population of granules. The granules as disclosed herein, can be prepared by any granulation method. As used herein, the term "granulation" refers to the process of agglomerating powder particles into larger agglomerates (i.e. granules) that contain the active agent. The term "granulation" includes dry and wet granulation techniques. The term "wet granulation" refers to any process comprising the steps of addition of a water comprising liquid, preferably water to the powder starting materials, preferably kneading, and drying to yield a solid dosage form. The term "dry granulation" refers to any process that comprises compacting the powder, usually either by slugging or with a roller compactor, and preferably milling the compacted powder to obtain the granules. No liquid is employed for the dry granulation. The compacted granulate or compacted granules as disclosed herein are prepared by dry granulation.

The term "extragranular component" as used herein refers to the set of ingredients in the pharmaceutical composition which have not been granulated and which do not form part of the granulate or granular component.

The term "pharmaceutically acceptable" indicates that the substance or composition must be compatible chemically and/or toxicologically, with the other ingredients comprising a formulation, and/or the mammal being treated therewith.

In another embodiment of the first aspect, the tablet comprises at least the following components as defined by the claims:
(a) a granulate which comprises at least a polyol and vildagliptin; and
(b) an extragranular component,
wherein the water content of the tablet is less than 10% w/w in respect of the total weight of the tablet, when measured by Karl Fischer titration.

As used herein, the term "polyol" means a pharmaceutically acceptable excipient which is an alcohol comprising multiple hydroxyl groups. Examples of polyols are sugar alcohols of general formula HOCH₂(CHOH)ₙCH₂OH, monosaccharides, disaccharides, polysaccharides and mixtures thereof. Non limiting examples of polyols as used herein include sugar alcohols such sorbitol, mannitol and xylitol; monosaccharides such glucose, fructose and galactose; disaccharides such as sucrose, lactose, trehalose and maltose; and polysaccharides such cellulose, dextrin, maltodrextrin and starch. Also known are polyols in form of a disaccharide, a polysaccharide or a mixture thereof, or the polyol can be a disaccharide selected from sucrose, lactose, trehalose, maltose and mixtures thereof. The best results were obtained when the polyol is lactose, which is according to the invention, preferably lactose anhydrous.

In another embodiment of the first aspect, the tablet according to any one of the preceding claims, wherein the water content of the tablet is between 0.1% and 9% w/w in respect of the total weight of the tablet when measured by Karl Fischer titration, and preferably wherein the water content of the tablet is between 0.1% and 5% w/w in respect of the total weight of the tablet when measured by Karl Fischer titration, more preferably the water content of the tablet is between 0.1% and 2% w/w in respect of the total weight of the tablet when measured by Karl Fischer titration.

In another embodiment of the first aspect, the tablet has a water content below 2 % w/w in respect of the total weight of the tablet, even after 7 days at 25 ºC/60 % RH and after 7 days at 40 ºC/75 % RH, when measured by Karl Fischer titration. Unless otherwise indicated, the conditions at 7 days and 25ºC/60% or 40ºC/75% as used herein can be both in open or close containers.

In another embodiment of the first aspect, the content of the polyol in the tablet is between 25 % and 80 % w/w in respect of the total weight of the tablet, more preferably the content of the polyol in the tablet is between 45% and 70 % w/w in respect of the total weight of the tablet. Preferably the polyol is a disaccharide selected from sucrose, lactose, maltose and mixtures thereof, more preferably the polyol is lactose anhydrous.

The pharmaceutical compositions as disclosed herein are stable. The term "stable" as used herein refers to a pharmaceutical composition comprising vildagliptin wherein the total content of impurities originated from the decomposition of vildagliptin does not exceed 5 % area, preferably 3 % area, more preferably 2 % area and most preferably 1 % area determined by liquid chromatography (HPLC) with UV detection at 210 nm if such a composition is stored for at least 2 months at 40°C and 75 % relative humidity (RH).

In another embodiment of the first aspect, the tablet is an immediate release tablet. The preferred tablet is therefore, not an orally disintegrating tablet nor an orally dissolving tablet, an effervescent tablet nor a modified release, controlled release nor sustained release tablet.

An immediate release tablet as herein disclosed has to be understood as a tablet having a dissolution performance such as 60 % or more of the active agent contained in said pharmaceutical composition dissolves within 60 minutes (min). In a preferred embodiment, the immediate release composition as herein disclosed releases at least 80 % of the active agent in 60 minutes. In another embodiment, the pharmaceutical composition as herein disclosed releases at least 80 % of the active agent in 45 min, preferably in 35 min and more preferably in 30 min. In another embodiment, the pharmaceutical composition as herein disclosed releases at least 80 % of the active agent in 30 min and at least a 95 % of the active agent in 60 min. In a most preferred embodiment, the pharmaceutical composition as herein disclosed releases at least 80 % of the active agent in 15 min, and at least 95 % of the active agent in 60 min. Preferably, the pharmaceutical composition releases at least 85 % of the active agent in 15 min. The dissolution test for an immediate release pharmaceutical composition comprising the active agent as herein disclosed is performed in the following conditions: Paddle. Speed: 50 rpm. Medium: 0.1 N HCl. Wavelength 210 nm.

In another embodiment of the first aspect, the granulate (a) is obtained by a process other than wet granulation or melt granulation. The granulate (a) is obtained by a process other than alcoholic wet granulation, aqueous moisture granulation or non-aqueous moisture granulation. The vildagliptin composition in the market, Galvus^{®}, is manufactured by direct compression.

The granulate (a) is obtained by dry granulation. Preferably, the granulate (a) is obtained by roller compaction.

In a preferred embodiment of the first aspect, the tablet comprises at least the following components as defined by the claims:
(a) a granulate which comprises lactose anhydrous and vildagliptin or a pharmaceutically acceptable salt thereof; and
(b) an extragranular component,

preferably the content of the polyol in the tablet is between 25 % and 80 % w/w in respect of the total weight of the tablet wherein the granulate (a) is obtained by dry granulation;
wherein the water content of the tablet is less than 10% w/w in respect of the total weight of the tablet, when measured by Karl Fischer titration.

In another embodiment of the first aspect, both tablet components are free of any glidant. Preferably, the tablet is free of talc or silica or any silicified microcrystalline cellulose. As used herein, "glidant" means a substance that improves the flowability of the pharmaceutical composition of the present invention when it is in form of a powder.

In another embodiment of the first aspect, the tablet is uncoated. In the tablet of the first aspect, neither the tablets nor the granules are coated.

In a preferred embodiment, the granulate (a) further comprises at least one lubricant.

Preferably, the lubricant selected from magnesium stearate, sodium stearyl fumarate, stearic acid, and mixtures thereof. More preferably, the lubricant is a hydrophilic surfactant selected from sodium stearyl fumarate, sodium lauryl sulfate, polyoxyl 40 stearate, polysorbate, polyoxyethylene lauryl ether, sorbitan monooleate, sodium oleate, sodium benzoate, sodium acetate, magnesium lauryl sulfate. The most preferred lubricant is sodium stearyl fumarate. Tablets comprising sodium stearyl fumarate present shorter disintegration times than with other lubricants such as magnesium stearate.

In a preferred embodiment, the tablet comprises sodium stearyl fumarate in both components, i.e both intragranular and extragranular components. As used herein, "lubricant" means a substance that reduces friction between the composition of the present invention and the surfaces of the apparatus used to compact the composition into a compressed form. In a preferred embodiment of the first aspect, the content of the lubricant in the tablet is between 0.1 % and 5% w/w in respect of the total weight of the tablet, preferably between 1% and 3% w/w in respect of the total weight of the tablet. Higher concentrations of lubricant could decrease tablet hardness and increase rate of dissolution. Preferably, the amount of lubricant in the tablet is divided into the two portions, intragranular and extragranular. More preferably, 50% of the lubricant is present in each of the portions.

In a preferred embodiment of the first aspect, the tablet comprises at least the following components as defined by the claims:
(a) a granulate which comprises lactose anhydrous and vildagliptin or a pharmaceutically acceptable salt thereof; and
(b) an extragranular component,

wherein the granulate (a) is obtained by dry granulation;
preferably the content of the polyol in the tablet is between 25 % and 80 % w/w in respect of the total weight of the tablet;
and wherein the granulate further comprises at least one lubricant, preferably the lubricant is a hydrophilic surfactant, more preferably the content of the lubricant in the tablet is between 0.1 % and 5% in respect of the total weight of the tablet;
preferably the water content of the tablet is less than 10% w/w in respect of the total weight of the tablet, when measured by Karl Fischer titration.

In a preferred embodiment of the first aspect, the tablet comprises at least the following components as defined by the claims:
(a) a granulate which comprises lactose anhydrous and vildagliptin or a pharmaceutically acceptable salt thereof; and
(b) an extragranular component,

the content of the polyol in the tablet is between 25 % and 80 % w/w in respect of the total weight of the tablet
wherein the granulate (a) is obtained by dry granulation;
and wherein the granulate further comprises at least one lubricant, the lubricant is sodium stearyl fumarate, preferably the content of the lubricant in the tablet is between 0.1 % and 5% in respect of the total weight of the tablet;
the water content of the tablet is less than 10% w/w in respect of the total weight of the tablet, when measured by Karl Fischer titration, more preferably the water content of the tablet is between 0.1% and 5% w/w in respect of the total weight of the tablet when measured by Karl Fisher titration.

In another embodiment of the first aspect, component (a) is free of polycarbophil, dibasic calcium phosphate, Polyethylene glycol (PEG), polyethylene oxide (PEG), carbomer, sodium alginate, alginic acid, Polyvinyl alcohol (PVA), poloxamer, and extended or sustained release excipients such as hydroxypropyl methylcellulose in at least 20% w/w in respect of the total weight of the tablet. In a preferred embodiment, the tablet is free of hydrophobic lubricants such as magnesium stearate. Tablets comprising hydrophobic lubricants such as magnesium stearate show slower dissolution rates.

The extragranular component (b) comprises a filler. Preferably, the tablet comprises less than 80 % w/w of filler of the extragranular component (b) in respect of the total weight of the tablet and/or at least 10 % w/w of the total amount of filler in the extragranular component (b) in respect of the total weight of the tablet.

The filler of the extragranular component (b) is microcrystalline cellulose. In a preferred embodiment of the first aspect, the tablet comprises at least the following components as defined by the claims:
(a) a granulate which comprises lactose anhydrous and vildagliptin or a pharmaceutically acceptable salt thereof; and
(b) an extragranular component,

the content of the polyol in the tablet is between 25 % and 80 % w/w in respect of the total weight of the tablet;
wherein the granulate (a) is obtained by dry granulation;
wherein the granulate further comprises at least one lubricant, preferably the lubricant is sodium stearyl fumarate, preferably the content of the lubricant in the tablet is between 0.1 % and 3% in respect of the total weight of the tablet; and
wherein the extragranular component (b) comprises a filler, preferably the filler is selected from microcrystalline cellulose, lactose, starch and mixtures thereof, more preferably the filler of the extragranular component (b) is microcrystalline cellulose; the water content of the tablet is less than 10% w/w in respect of the total weight of the tablet, when measured by Karl Fischer titration.

In a preferred embodiment of the first aspect, the tablet comprises at least the following components as defined by the claims:
(a) a granulate which comprises lactose anhydrous and vildagliptin or a pharmaceutically acceptable salt thereof; and
(b) an extragranular component,

wherein the content of the polyol in the tablet is between 25 % and 80 % w/w in respect of the total weight of the tablet;
wherein the granulate (a) is obtained by dry granulation;
wherein the granulate further comprises at least one lubricant, the lubricant is sodium stearyl fumarate, preferably the content of the lubricant in the tablet is between 0.1 % and 3% in respect of the total weight of the tablet; and
wherein the extragranular component (b) comprises a filler, the filler is microcrystalline cellulose, preferably the tablet comprises less than 80 % w/w of filler of the extragranular component (b) in respect of the total weight of the tablet and/or at least 10 % w/w of the total amount of filler in the extragranular component (b) in respect of the total weight of the tablet;
the water content of the tablet is less than 10% w/w in respect of the total weight of the tablet, when measured by Karl Fischer titration.

A higher percentage of microcrystalline cellulose has an impact in the dissolution performance of the tablets.

The term "filler" as used herein refers to pharmaceutically acceptable excipients which are added to the bulk volume of the active agent making up the solid composition. As a result, the size of the solid composition increases, which makes its size suitable for handling. Fillers are convenient when the dose of drug per solid composition is low and the solid composition would otherwise be too small.

The polyol is selected from lactose anhydrous and mixtures with microcrystalline cellulose anhydrous. In another embodiment the polyol is a disaccharide. More preferably, the polyol is lactose anhydrous. In another embodiment, the granulate (a) does not comprise microcrystalline cellulose. The percentage of total impurities decreases as the percentage of anhydrous lactose increases in respect to the total amount of intragranular diluent. Formulations comprising high percentages of lactose anhydrous intragranularly present the lowest impurity increase. More preferably, the only diluent present in the granulate is lactose anhydrous.

The tablet further comprises at least a disintegrant agent. Preferably, the tablet further comprises sodium croscarmellose. In a preferred embodiment, the content of the disintegrant in the tablet is between 0.5 % and 5 % w/w in respect of the total weight of the tablet.

As used herein, "disintegrant" means a substance or a mixture of substances added to a tablet to facilitate its breakup or disintegration after administration.

In a preferred embodiment of the first aspect, the tablet comprises at least the following components as defined by the claims:
(a) a granulate which comprises lactose anhydrous and vildagliptin or a pharmaceutically acceptable salt thereof; and
(b) an extragranular component,

the content of the polyol in the tablet is between 25 % and 80 % w/w in respect of the total weight of the tablet
wherein the granulate (a) is obtained by dry granulation;
wherein the granulate further comprises at least one lubricant, the lubricant is sodium stearyl fumarate, preferably the content of the lubricant in the tablet is between 0.1 % and 3% in respect of the total weight of the tablet; and
wherein the tablet further comprises at least a disintegrant agent, preferably the disintegrant agent is sodium croscarmellose;
the water content of the tablet is less than 10% w/w in respect of the total weight of the tablet, when measured by Karl Fischer titration.

Tablets comprising these technical features have shown improved stability.

In a preferred embodiment of the first aspect, the tablet comprises at least the following components as defined by the claims:
(a) a granulate which comprises lactose anhydrous and vildagliptin or a pharmaceutically acceptable salt thereof; and
(b) an extragranular component,

wherein the granulate (a) is obtained by dry granulation;
wherein the content of the polyol in the tablet is between 25 % and 80 % w/w in respect of the total weight of the tablet;
wherein the granulate further comprises at least one lubricant, preferably the lubricant is sodium stearyl fumarate, more preferably the content of the lubricant in the tablet is between 0.1 % and 3% in respect of the total weight of the tablet;
wherein the extragranular component (b) comprises a filler, preferably the filler of the extragranular component (b) is microcrystalline cellulose, more preferably the tablet comprises less than 80 % w/w of filler of the extragranular component (b) in respect of the total weight of the tablet and/or at least 10 % w/w of the total amount of filler in the extragranular component (b) in respect of the total weight of the tablet; and wherein the tablet further comprises at least a disintegrant agent, preferably the disintegrant agent is sodium croscarmellose, more preferably the content of the disintegrant in the tablet is between 0.5 % and 5 % w/w in respect to the total weight of the tablet;
the water content of the tablet is less than 10% w/w in respect of the total weight of the tablet, when measured by Karl Fischer titration.

In a preferred embodiment of the first aspect, the tablet comprises at least the following components as defined by the claims:
(a) a granulate which comprises lactose anhydrous and vildagliptin or a pharmaceutically acceptable salt thereof; and
(b) an extragranular component,

wherein the granulate (a) is obtained by dry granulation;
wherein the granulate further comprises at least one lubricant, the lubricant is sodium stearyl fumarate, more preferably the content of the lubricant in the tablet is between 0.1 % and 3% in respect of the total weight of the tablet;
wherein the extragranular component (b) comprises a filler, the filler is microcrystalline cellulose, preferably the tablet comprises less than 80 % w/w of filler of the extragranular component (b) in respect of the total weight of the tablet and/or at least 10 % w/w of the total amount of filler in the extragranular component (b) in respect of the total weight of the tablet; and
wherein the tablet further comprises at least a disintegrant agent, the disintegrant agent is sodium croscarmellose, preferably the content of the disintegrant in the tablet is between 0.5 % and 5 % w/w in respect to the total weight of the tablet;
the water content of the tablet is less than 10% w/w in respect of the total weight of the tablet, when measured by Karl Fischer titration.

In a preferred embodiment of the first aspect, the tablet comprises at least the following components as defined by the claims:
(a) a granulate which comprises lactose anhydrous and vildagliptin or a pharmaceutically acceptable salt thereof; and
(b) an extragranular component,

wherein the content of lactose in the tablet is between 25 % and 80 % w/w in respect of the total weight of the tablet;
wherein the granulate (a) is obtained by dry granulation;
wherein the granulate further comprises at least one lubricant, the lubricant is sodium stearyl fumarate, the content of the lubricant in the tablet is between 0.1 % and 3% in respect of the total weight of the tablet;
wherein the extragranular component (b) comprises a filler, the filler is microcrystalline cellulose, the tablet comprises less than 80 % w/w of filler of the extragranular component (b) in respect of the total weight of the tablet and/or at least 10 % w/w of the total amount of filler in the extragranular component (b) in respect of the total weight of the tablet; and
wherein the tablet further comprises at least a disintegrant agent, the disintegrant agent is sodium croscarmellose, the content of the disintegrant in the tablet is between 0.5 % and 5 % w/w in respect to the total weight of the tablet;
the water content of the tablet is less than 10% w/w in respect of the total weight of the tablet, when measured by Karl Fischer titration.

In another embodiment of the first aspect, the tablet further comprises at least one active agent, wherein the active agent is an antidiabetic drug. Preferably the active agent is selected from metformin, a sulfonylurea, a thiazolidinedione and mixtures thereof, more preferably the tablet comprises metformin.

In a preferred embodiment, the tablet further comprises metformin as a granulated, wherein the vildagliptin and the metformin are granulated separately.

In another preferred embodiment, the tablet further comprises metformin as a granulated, wherein the granulated comprises both vildagliptin and metformin.

In another preferred embodiment, the tablet further comprises metformin as a granulated, wherein the granulated comprises both vildagliptin and metformin and wherein the polyol is microcrystalline cellulose.

In a preferred embodiment of the first aspect, vildagliptin is the only active agent in the tablet.

A preferred embodiment of the first aspect is an immediate release tablet consisting of a dry granulate of vildagliptin, a polyol and at least one pharmaceutically acceptable excipient, and at least one extragranular pharmaceutically acceptable excipient, wherein the water content of the tablet is less than 10 % w/w in respect of the total weight of the tablet, when measured by Karl Fischer titration.

Preferably, the water content of the tablet is less than 10% w/w in respect of the total weight of the tablet, when measured by Karl Fischer titration after 1 month at 40 ºC and 75% relative humidity.

A second aspect not according to the claimed invention is a pharmaceutical package which comprises at least one tablet according to the first aspect. Preferably, the pharmaceutical package comprises at least one blister pack comprising at least one tablet according to the first aspect, and preferably the blister pack is of aluminium/PVC or aluminium/aluminium. More preferably, the pharmaceutical package is in the form of a cardboard box.

The term "blister" or bubble pack refers to a sheet in a package construction with recesses designed to hold dosage forms. The sheet may be a plastic, a foil, or combination thereof. Normally, a blister is a product consisting of a flat structure in which blisters are formed, generally by means of a heating process, into which the single elements to be packaged are inserted. The blisters are then hermetically sealed using flat strips of appropriate thermoformable materials (plastics, aluminum, paper), which represent the frangible element through which it is then possible to remove the product. The primary component of a blister pack is a cavity or pocket made from a formable web, usually a thermoformed plastic. This usually has a backing of paperboard or a lidding seal of aluminum foil or plastic. An aluminum/PVC blister refers to a blister where the thermoformable material is from PVC and the backing is a lidding seal of aluminum foil. A PVC/PE/PVDC/aluminum blister refers to a blister where the thermoformable material is a laminate of PVC/PE/PVDC and the backing is a lidding seal of aluminum foil. PVC is poly(vinylchloride), PE is polyethylene and PVDC is polyvinylidene chloride. Blister packs are commonly used as unit-dose packaging for pharmaceutical tablets, capsules or lozenges. Blister packs can provide barrier protection for shelf life requirements, and a degree of tamper resistance. In the USA, blister packs are mainly used for packing physician samples of drug products, or for Over The Counter (OTC) products in the pharmacy. In other parts of the world, blister packs are the main packaging type since pharmacy dispensing and repackaging are not common. A series of blister cavities is sometimes called a blister card or blister strip as well as blister pack. The difference between a strip pack and blister pack is that a strip pack does not have thermo-formed or cold formed cavities; the strip pack is formed around the tablet at a time when it is dropped to the sealing area between sealing moulds. In some parts of the world the pharmaceutical blister pack is known as a Push-Through-Pack (PTP), an accurate description of two key properties (i) the lidding foil is brittle allowing to press the product out while breaking the lidding foil and (ii) a semi-rigid formed cavity being sufficiently collapsible to be able to dispense the tablet or capsule by means of pressing it out with your thumb.

The main advantages of unit-dose blister packs over other methods of packing pharmaceutical products are the assurance of product/packaging integrity (including shelflife) of each individual dose and the possibility to create a compliance pack or calendar pack by printing the days of the week above each dose. Blister packs can be created by means of a form-fill-seal process at the pharmaceutical company or designated contract packer. A form-fill-seal process means that the blister pack is created from rolls of flat sheet or film, filled with the pharmaceutical product and closed (sealed) on the same equipment. Such equipment is called a blisterline. There are two types of blister machine's design: rotary and flat-plate.

As used herein, the term "unit dose" or "unit dosage" refers to a physically discrete unit that contains a predetermined quantity of active agent calculated to produce a desired therapeutic effect. The unit dose or unit dosage may be in the form of a tablet, capsule, sachet, etc. referred to herein as a "unit dosage form".

A third aspect not according to the claimed invention is a pharmaceutical batch of at least 50,000 tablets, preferably of at least 100,000 tablets according to the first aspect, wherein the vildagliptin content or the pharmaceutically acceptable salt thereof content is uniform and/or the tablets are packaged in a blister pack of aluminium/PVC or aluminium/aluminium.

The term "batch" as used herein refers to a specific quantity of a drug or other material that is intended to have uniform character and quality, within specified limits, and is produced according to a single manufacturing order during the same cycle of manufacture. A batch, in the case of a drug product produced by continuous process, is a specific identified amount produced in a unit of time or quantity in a manner that assures its having uniform character and quality within specified limits (Code of Federal Regulations Title 21, Food and Drug Administration department of Health and Human Services, Subchapter C, Section 210.3 (b) (2) and (10)).

The term "pharmaceutical batch" as used herein refers to a batch as defined above of a pharmaceutical composition manufactured in accordance with the principles and guidelines of Good Manufacturing Practice (GMP) at an industrial scale and which is intended for commercialization (Directive 91/356/EEC).

The pharmaceutical composition may be manufactured at laboratory scale, not necessarily following GMP and not intended for commercialization. The pharmaceutical composition may also be manufactured for validation, following GMP.

A batch of a pharmaceutical composition which is manufactured for validation is called "pilot batch".

Each pharmaceutical batch of finished product must fulfil the regulatory requirements of the corresponding Medicine Agency before being released for sale or supply, such as impurities thresholds and stability data.

The term "uniform" as used herein refers to the content of the active agent in the tablets of a pharmaceutical batch has to be homogeneous. According to the FDA criteria, uniformity is considered as achieving 90-110 % potency of the theoretical strength with a relative standard deviation (RSD) of less than 5 % for all samples (Guidance for Industry ANDA's: Blend Uniformity Analysis, published August 1999).

The term "active agent" refers to a therapeutically active compound, as well as any prodrugs thereof and pharmaceutically acceptable salts, hydrates and solvates of the compound and the prodrugs.

For the release of a pharmaceutical batch the distribution of the active agent in the tablets has to be homogeneous, that is, content uniformity is required. All batches are expected to be uniform within normal process variation. Process validation studies are conducted prior to the marketing of a drug product to assure that production processes are controlled. The test batch is manufactured prior to validation, yet it is the basis on which an application is approved (MANUAL OF POLICIES AND PROCEDURES, MAPP 5225.1).

It is essential, therefore, to assure that the test batch is uniform. In-process tests for uniformity should be conducted throughout the entire production process, e.g., at commencement or completion of significant phases (21 CFR 211.110). These tests should be designed to detect potential in-process anomalies (MAPP 5225.1).

A fourth aspect of the invention is a process for the manufacture of the tablet according to the first aspect, comprising the following steps as defined by the claims:
(i) weighing, sieving and mixing at least a polyol, vildagliptin and optionally at least one pharmaceutically acceptable excipient, and
(ii) optionally dry granulating the mix obtained in step (i),
(iii) blending the dry granulate obtained in step (ii) with at least one extragranular pharmaceutically acceptable excipient, and
(iv) compressing the mix obtained in step (iii) to form a tablet.

In a preferred embodiment of the fourth aspect, the process does not comprise a step of drying the granules.

The polyol comprises lactose more preferably the polyol is lactose anhydrous.

In another embodiment of the fourth aspect, a dry granulation takes place in step (ii).

Preferably, the dry granulation of step (ii) is carried out by roller compaction. After the roller compaction there is a step of milling the compacted powder to obtain granules.

In another embodiment of the fourth aspect, step (i) further comprises a lubricant.

Accordingly, the mixture obtained in step (i) preferably comprises at least a polyol, vildagliptin and a lubricant. Preferably the lubricant is a hydrophilic surfactant, more preferably the lubricant is sodium stearyl fumarate.

In another embodiment of the fourth aspect, step (i) further comprises a lubricant wherein the lubricant is sodium stearyl fumarate and the content of the lubricant in the mixture of step (i) is between 0.05% and 2.5% w/w in respect of the total weight of the tablet, preferably between 0.5% and 1.5% w/w in respect of the total weight of the tablet.

In another embodiment of the fourth aspect, the at least one extragranular pharmaceutically acceptable excipient of step (iii) comprises a filler which is microcrystalline cellulose.

In another embodiment of the fourth aspect, a dry granulation takes place in step (ii), wherein the dry granulation of step (ii) is carried out by roller compaction;
the mixture of step (i) further comprises a lubricant, preferably the lubricant is sodium stearyl fumarate; the content of the lubricant in the mixture of step (i) is between 0.05% and 2.5% w/w in respect of the total weight of the tablet; and
the at least one extragranular pharmaceutically acceptable excipient of step (iii) comprises a filler wherein the filler comprises microcrystalline cellulose.

In another embodiment of the fourth aspect, the at least one extragranular pharmaceutical acceptable excipient of step (iii) comprises a disintegrant, preferably the disintegrant is sodium croscarmellose. Preferably the content of disintegrant in the tablet is between 0.5% and 5% w/w in respect to the total weight of the tablet.

In another embodiment of the fourth aspect, the at least one extragranular pharmaceutical acceptable excipient of step (iii) comprises a lubricant and a disintegrant;
wherein the lubricant is preferably sodium stearyl fumarate; and
wherein the disintegrant is preferably sodium croscarmellose.

In another embodiment of the fourth aspect, the at least one extragranular pharmaceutical acceptable excipient of step (iii) comprises a filler, a lubricant and a disintegrant;
wherein the filler is microcrystalline cellulose;
wherein the lubricant is sodium stearyl fumarate and the content of the lubricant in the mixture of step (i) is preferably between 0.05% and 2.5% w/w in respect of the total weight of the tablet; and
wherein the disintegrant is preferably sodium croscarmellose and the content of disintegrant in the tablet is between 0.5% and 5% w/w in respect to the total weight of the tablet.

A fifth aspect not according to the claimed invention is a tablet manufactured according to the process of the present invention.

A sixth aspect not according to the claimed invention is a process for the manufacture of a pharmaceutical batch of a tablet of the first aspect, wherein the process comprises the following steps:
(i) preparing a test tablet of vildagliptin or a pharmaceutically acceptable salt thereof;
(ii) checking the stability and dissolution profile of the test tablet of step (i); and
(iii) manufacturing a pharmaceutical batch by the same manufacturing process used to prepare the test tablet in step (i) provided that the test tablet is stable and has an immediate release dissolution profile; and
(iv) optionally, packaging the pharmaceutical batch manufactured in step (iii) preferably in blister packs or in bottles.

A seventh aspect not according to the claimed invention is a pharmaceutical batch validated by a process comprising the following steps:
i) manufacturing the pharmaceutical batch;
ii) checking the uniformity of the vildagliptin or pharmaceutical acceptable salt thereof content; and
iii) validating the batch only if the content is uniform.

An eighth aspect of the invention is the tablet of the first aspect, for use in the treatment of type 2 diabetes mellitus.

All percentages, parts, and ratios herein are by weight unless specifically noted otherwise. As used herein, the term "about" refers preferably to a range that is ±10 %, preferably ±5 %, or more preferably ±1 % of a value with which the term is associated.

Unless otherwise indicated, all the analysis methods are carried out according to the European Pharmacopoeia 7th edition.

In the context of the invention, the Karl Fischer titration is carried out according to methods known in the field of technology, the European Pharmacopoeia 7th Ed. The water content is determined according to the test described in *Ph. Eur.* (2.5.12) by semimicro determination method (KF). Examine by Karl Fischer volumetric titration.

Tablets were finely pulverizing and an accurately weighed amount was transferred to a Karl Fisher titration vessel and titrated using a suitable reagent for aldehydes and ketones.

### EXAMPLES

### Example 1. Tablet compositions:

The vildagliptin used in these tablets was crystalline vildagliptin of form A as disclosed in WO2006078593. The vildagliptin particles used showed a particle size distribution having a D10 of 30 microns, a D50 of 145 microns and a D90 of 425 microns, analysed by laser diffraction spectroscopy using a Malvern Mastersizer 2000 particle size analyser.

| **Components** | **1A mg/tablet** | **1B mg/tablet** | **1C mg/tablet** | **1D mg/tablet** | **1E mg/tablet** |
|---|---|---|---|---|---|
| *Intragranular* | | | | | |
| Vildagliptin | 50 | 50 | 50 | 50 | 50 |
| Lactose anhydrous | 30 | 100 | 50 | 150 | 20 |
| Microcrystalline cellulose | - | - | 45 | - | - |
| Sodium stearyl fumarate | 0.5 | 2 | 2 | 0.5 | 4 |

| *Extragranular* | | | | | |
|---|---|---|---|---|---|
| Microcrystalline cellulose | 113 | 40 | 46.5 | 30 | 116 |
| Croscarmellose sodium | 6 | 6 | 6 | 6 | 6 |
| Sodium stearyl fumarate | 0.5 | 2 | 0.5 | 0.5 | 4 |

Batches of 100,000 tablets were manufactured for the compositions of example 1. The manufacturing process was as follows: the vildagliptin and intragranular excipients were sieved and mixed. The resulting mix was compressed in a roller compactor and the obtained product was milled to obtain a granulate. The granulate was mixed with the previously sieved extragranular excipients except for the lubricant, which was sieved and added before mixing and compressing the final mix into tablets. The tablets were packaged in aluminium/PVC or Alu/Alu blisters and said blisters were packaged in cardboard boxes.

### Example 2. Stability.

Tablets of example 1 are subjected to stability tests at times 0, 15 days, 1 month and 2 months at 40 ºC and 75 % Relative Humidity (RH).

Percentage of impurities is determined by HPLC (Column XBridge C8, flow rate 1.2 ml/min, UV detector wavelength 210nm, column temperature 35ºC, auto-sampler temperature 5ºC, Retention time (RT) vildagliptin 20 min, mobile phase A: acetonitrile with gradient. Mobile phase A:900 ml buffer pH 8 with 100 ml of acetonitrile. RT of impurities at 3.9 min, 4.9 min, 5.8 min, 7.5 min, 16.8 min and 29.0 min.

### Example 3. Water content.

The water content of the tablets of example 1 was calculated by Karl Fischer titration as described above. All tablets had a water content below 1 % w/w in respect of the total weight of the tablet at time 0. After 8 days at 25 ºC/60 % RH or at 40 ºC/75 % RH, the water content of the tablets was still below 2 % w/w in respect of the total weight of the tablet.

### Example 4. Dissolution profile.

The tablets of example 1 have an immediate release dissolution profile. The dissolution profile was tested in a paddle apparatus at 50 rpm and with 0.1 N HCl as dissolution medium in a volume of 900 ml per vessel at a temperature of 37± 0.5 ºC. Each tablet is weighted individually and one unit is placed in each vessel. Determination of vildagliptin is carried out by HPLC (Column XBridge C8, flow rate 0.8 ml/min, UV detector wavelength 210 nm, column temperature 40ºC, auto-sampler temperature RT aprox 22ºC, RT vildagliptin 3.6 min, mobile phase buffer pH 4.5:acetonitrile (85:15). In all the compositions of the examples, more than 80 % of the vildagliptin was dissolved in 20 minutes.

## Claims

1. - A tablet, preferably an immediate release tablet, which comprises at least the following components:
(a) a granulate which comprises at least a polyol and vildagliptin or a pharmaceutically acceptable salt thereof; and
(b) an extragranular component comprising a filler,
wherein the water content of the tablet is less than 10% w/w in respect of the total weight of the tablet, when measured by Karl Fischer titration, preferably wherein the water content of the tablet is between 0.1% and 9% w/w in respect of the total weight of the tablet when measured by Karl Fischer titration, and preferably wherein the water content of the tablet is between 0.1% and 5% w/w in respect of the total weight of the tablet when measured by Karl Fischer titration, wherein the granulate is obtained by dry granulation, wherein the filler is microcrystalline cellulose, wherein the polyol is lactose and wherein the tablet further comprises at least a disintegrant agent.

2. The tablet according to the preceding claim wherein the lactose comprised in the granulate is anhydrous.

3. The tablet according to the preceding claim wherein the lactose content in the granulate is between 25% w/w and 80% w/w in respect of the total weight of the tablet, more preferably the content of lactose in the tablet is between 45% and 70 % w/w in respect of the total weight of the tablet.

4. - The tablet according to any one of the preceding claims, wherein the granulate (a) is obtained by roller compaction.

5. - The tablet according to any one of preceding claims, wherein the granulate (a) further comprises at least one lubricant, preferably further comprising at least one lubricant selected from magnesium stearate, sodium stearyl fumarate, stearic acid, and mixtures thereof, preferably wherein the lubricant is sodium stearyl fumarate, preferably the content of the lubricant in the tablet is between 0.1 % and 3% w/w in respect of the total weight of the tablet.

6. - The tablet according to any one of the preceding claims, wherein the extragranular component (b) comprises a filler, wherein the filler of the extragranular component (b) is microcrystalline cellulose, which comprises less than 80 % w/w of filler of the extragranular component (b) in respect of the total weight of the tablet and/or at least 10 % w/w of the total amount of filler in the extragranular component (b) in respect of the total weight of the tablet.

7. - The tablet according to any one of the preceding claims, wherein the disintegrant agent is sodium croscarmellose, preferably the content of the disintegrant in the tablet is between 0.5 % and 5 % w/w in respect to the total weight of the tablet.

8. - The tablet according to any one of the preceding claims, wherein vildagliptin is the only active agent or the tablet according to any one of claims 1 to 7, further comprising another antidiabetic drug selected from metformin, a sulfonylurea, a thiazolidinedione and mixtures thereof, preferably metformin.

9. - A process for the manufacture of the tablet according to any one of the claims 1 to 8, comprising the following steps:
(i) weighing, sieving and mixing at least a polyol, vildagliptin and optionally at least one pharmaceutically acceptable excipient, preferably the mixture further comprises a lubricant, preferably the lubricant is a hydrophilic surfactant, more preferably the lubricant is sodium stearyl fumarate, and
(ii) dry granulating the mix obtained in step (i), preferably the dry granulation is carried out by roller compaction,
(iii) blending the dry granulate obtained in step (ii) with at least one extragranular pharmaceutically acceptable excipient, wherein the extragranular pharmaceutically acceptable excipients of step (iii) comprises a filler selected from microcrystalline cellulose, and a disintegrant, and
(iv) compressing the mix obtained in step (iii) to form a tablet.

10. - The process according to claim 9, wherein the extragranular pharmaceutically acceptable excipients of step (iii) comprises sodium croscarmellose or wherein the extragranular pharmaceutically acceptable excipients of step (iii) comprises a lubricant, preferably the lubricant is a hydrophilic surfactant, more preferably the lubricant is sodium stearyl fumarate.

11. - The tablet according to any one of claims 1 to 8, for use in the treatment of type 2 diabetes mellitus.

## Patentansprüche

1. Tablette, vorzugsweise eine Tablette mit sofortiger Freisetzung, die mindestens die folgenden Komponenten umfasst:
(a) ein Granulat, das mindestens ein Polyol und Vildagliptin oder ein pharmazeutisch verträgliches Salz davon umfasst; und
(b) eine extragranuläre Komponente, die einen Füllstoff umfasst,
wobei der Wassergehalt der Tablette bei Messung mittels Karl-Fischer-Titration weniger als 10 Gew.-%, bezogen auf das Gesamtgewicht der Tablette, beträgt, wobei der Wassergehalt der Tablette bei Messung mittels Karl-Fischer-Titration vorzugsweise zwischen 0,1 und 9 Gew.-%, bezogen auf das Gesamtgewicht der Tablette, liegt und wobei der Wassergehalt der Tablette bei Messung mittels Karl-Fischer-Titration vorzugsweise zwischen 0,1 und 5 Gew.-%, bezogen auf das Gesamtgewicht der Tablette, liegt, wobei das Granulat durch Trockengranulation erhalten wird, wobei der Füllstoff mikrokristalline Zellulose ist, wobei das Polyol Laktose ist und wobei die Tablette weiter mindestens ein Sprengmittel umfasst.

2. Tablette nach dem vorhergehenden Anspruch, wobei die von dem Granulat umfasste Laktose wasserfrei ist.

3. Tablette nach dem vorhergehenden Anspruch, wobei der Laktosegehalt in dem Granulat zwischen 25 Gew.-% und 80 Gew.-%, bezogen auf das Gesamtgewicht der Tablette, liegt, wobei der Laktosegehalt in der Tablette noch bevorzugter zwischen 45 und 70 Gew.-%, bezogen auf das Gesamtgewicht der Tablette, liegt.

4. Tablette nach einem der vorhergehenden Ansprüche, wobei das Granulat (a) durch Walzenverdichtung erhalten wird.

5. Tablette nach einem der vorhergehenden Ansprüche, wobei das Granulat (a) weiterhin mindestens ein Schmiermittel umfasst, vorzugsweise weiter umfassend mindestens ein Schmiermittel, das ausgewählt ist aus Magnesiumstearat, Natriumstearylfumarat, Stearinsäure und Mischungen davon, wobei das Schmiermittel vorzugsweise Natriumstearylfumarat ist, wobei der Schmiermittelgehalt in der Tablette vorzugsweise zwischen 0,1 und 3 Gew.-%, bezogen auf das Gesamtgewicht der Tablette, liegt.

6. Tablette nach einem der vorhergehenden Ansprüche, wobei die extragranuläre Komponente (b) einen Füllstoff umfasst, wobei der Füllstoff der extragranulären Komponente (b) mikrokristalline Zellulose ist, die weniger als 80 Gew.-% des Füllstoffs der extragranulären Komponente (b), bezogen auf das Gesamtgewicht der Tablette, und/oder mindestens 10 Gew.-% der Gesamtmenge an Füllstoff in der extragranulären Komponente (b), bezogen auf das Gesamtgewicht der Tablette, umfasst.

7. Tablette nach einem der vorhergehenden Ansprüche, wobei das Sprengmittel Natriumcroscarmellose ist, wobei der Sprengmittelgehalt in der Tablette vorzugsweise zwischen 0,5 und 5 Gew.-%, bezogen auf das Gesamtgewicht der Tablette, liegt.

8. Tablette nach einem der vorhergehenden Ansprüche, wobei Vildagliptin der einzige Wirkstoff ist, oder Tablette nach einem der Ansprüche 1 bis 7, weiter umfassend ein anderes Antidiabetikum, ausgewählt aus Metformin, einem Sulfonylharnstoff, einem Thiazolidindion und Mischungen davon, vorzugsweise Metformin.

9. Verfahren zur Herstellung der Tablette nach einem der Ansprüche 1 bis 8, umfassend die folgenden Schritte:
(i) Wiegen, Sieben und Mischen von mindestens einem Polyol, Vildagliptin und wahlweise mindestens einem pharmazeutisch verträglichen Hilfsstoff, wobei die Mischung vorzugsweise weiter ein Schmiermittel umfasst, wobei das Schmiermittel vorzugsweise ein hydrophiles Tensid ist, wobei das Schmiermittel noch bevorzugter Natriumstearylfumarat ist, und
(ii) Trockengranulieren des in Schritt (i) erhaltenen Gemisches, wobei die Trockengranulation vorzugsweise durch Walzenverdichtung erfolgt,
(iii) Mischen des in Schritt (ii) erhaltenen Trockengranulats mit mindestens einem extragranulären pharmazeutisch verträglichen Hilfsstoff, wobei die extragranulären pharmazeutisch verträglichen Hilfsstoffe von Schritt (iii) einen Füllstoff, ausgewählt aus mikrokristalliner Zellulose, und ein Sprengmittel umfasst, und
(iv) Verdichten des in Schritt (iii) erhaltenen Gemisches, um eine Tablette zu bilden.

10. Verfahren nach Anspruch 9, wobei die extragranulären pharmazeutisch verträglichen Hilfsstoffe von Schritt (iii) Natriumcroscarmellose umfasst oder wobei die extragranulären pharmazeutisch verträglichen Hilfsstoffe von Schritt (iii) ein Schmiermittel umfasst, wobei das Schmiermittel vorzugsweise ein hydrophiles Tensid ist, wobei das Schmiermittel noch bevorzugter Natriumstearylfumarat ist.

11. Tablette nach einem der Ansprüche 1 bis 8 zur Verwendung bei der Behandlung von Diabetes mellitus Typ 2.

## Revendications

1. Comprimé, de préférence comprimé à libération immédiate, qui comprend au moins les composants suivants :
(a) un granulé qui comprend au moins un polyol et de la vildagliptine ou un sel pharmaceutiquement acceptable de celle-ci ; et
(b) un composant extragranulaire comprenant un agent de charge,
dans lequel la teneur en eau du comprimé est inférieure à 10 % p/p par rapport au poids total du comprimé, lorsqu'elle est mesurée par titrage Karl Fischer, de préférence dans lequel la teneur en eau du comprimé est entre 0,1 % et 9 % p/p par rapport au poids total du comprimé lorsqu'elle est mesurée par titrage Karl Fischer, et de préférence dans lequel la teneur en eau du comprimé est entre 0,1 % et 5 % p/p par rapport au poids total du comprimé lorsqu'elle est mesurée par titrage Karl Fischer, dans lequel le granulé est obtenu par granulation à sec, dans lequel l'agent charge est de la cellulose microcristalline, dans lequel le polyol est du lactose et dans lequel le comprimé comprend en outre au moins un agent désintégrant.

2. Comprimé selon la revendication précédente dans lequel le lactose compris dans le granulé est anhydre.

3. Comprimé selon la revendication précédente dans lequel la teneur en lactose dans le granulé est entre 25 % p/p et 80 % p/p par rapport au poids total du comprimé, plus préférentiellement la teneur en lactose dans le comprimé est entre 45 % et 70 % p/p par rapport au poids total du comprimé.

4. Comprimé selon l'une quelconque des revendications précédentes, dans lequel le granulé (a) est obtenu par compactage au rouleau.

5. Comprimé selon l'une quelconque des revendications précédentes, dans lequel le granulé (a) comprend en outre au moins un lubrifiant, de préférence comprenant en outre au moins un lubrifiant choisi parmi le stéarate de magnésium, le stéarylfumarate de sodium, l'acide stéarique, et des mélanges de ceux-ci, de préférence dans lequel le lubrifiant est le stéarylfumarate de sodium, plus préférentiellement la teneur en lactose dans le comprimé est entre 0,1 % et 3 % p/p par rapport au poids total du comprimé.

6. Comprimé selon l'une quelconque des revendications précédentes, dans lequel le composant extragranulaire (b) comprend un agent de charge, dans lequel l'agent de charge du composant extragranulaire (b) est de la cellulose microcristalline, qui comprend moins de 80 % p/p de charge du composant extragranulaire (b) par rapport au poids total du comprimé et/ou au moins 10 % p/p de la quantité totale d'agent charge dans le composant extragranulaire (b) par rapport au poids total du comprimé.

7. Comprimé selon l'une quelconque des revendications précédentes, dans lequel l'agent désintégrant est de la croscarmellose sodique, plus préférentiellement la teneur en désintégrant dans le comprimé est entre 0,5 % et 5 % p/p par rapport au poids total du comprimé.

8. Comprimé selon l'une quelconque des revendications précédentes, dans lequel la vildagliptine est le seul principe actif ou le comprimé selon l'une quelconque des revendications 1 à 7, comprenant en outre un autre médicament antidiabétique choisi parmi la metformine, une sulfonylurée, une thiazolidinedione et des mélanges de celles-ci, de préférence la metformine.

9. Procédé de fabrication du comprimé selon l'une quelconque des revendications 1 à 8, comprenant les étapes suivantes :
(i) peser, tamiser et mélanger au moins un polyol, de la vildagliptine et éventuellement au moins un excipient pharmaceutiquement acceptable, de préférence le mélange comprend en outre un lubrifiant, de préférence le lubrifiant est un tensioactif hydrophile, plus préférentiellement, le lubrifiant est du stéarylfumarate de sodium, et
(ii) granuler à sec le mélange obtenu à l'étape (i), de préférence la granulation à sec est réalisée par compactage au rouleau,
(iii) mélanger le granulé sec obtenu à l'étape (ii) avec au moins un excipient extragranulaire pharmaceutiquement acceptable, dans lequel les excipients extragranulaires pharmaceutiquement acceptables de l'étape (iii) comprend un agent de charge choisi parmi la cellulose microcristalline et un désintégrant, et
(iv) compresser le mélange obtenu à l'étape (iii) pour former un comprimé.

10. Procédé selon la revendication 9, dans lequel les excipients extragranulaires pharmaceutiquement acceptables de l'étape (iii) comprend de la croscarmellose sodique ou dans lequel les excipients extragranulaires pharmaceutiquement acceptables de l'étape (iii) comprend un lubrifiant, de préférence le lubrifiant est un tensioactif hydrophile, plus préférentiellement, le lubrifiant est du stéarylfumarate de sodium.

11. Comprimé selon l'une quelconque des revendications 1 à 8, pour une utilisation dans le traitement du diabète sucré de type 2.
